# EUROPÄISCHE PATENTSCHRIFT

(11) **EP 1 331 033 B1**
(45) Veröffentlichungstag und Bekanntmachung des Hinweises auf die Patenterteilung: **14.03.2007**
(21) Anmeldenummer: 03001147.2
(22) Anmeldetag: 21.01.2003
(51) Int. Cl.: B01J 35/08, B01J 25/00, B01J 2/08, B01J 2/28, C10G 45/46

(54) **Kugelförmige hochaktive Metall-Trägerkatalysatoren**
Highly active spherical metal support catalysts
Catalyseurs fortement actifs avec support contenant une composante métallique

(30) Priorität: 22.01.2002 DE 10202127
(43) Veröffentlichungstag der Anmeldung: 30.07.2003
(73) Patentinhaber: SHELL INTERNATIONALE RESEARCH MAATSCHAPPIJ B.V., 2596 HR Den Haag (NL)
(72) Erfinder:

(56) Entgegenhaltungen:
- EP-A- 0 479 553
- CH-A- 566 809
- DE-A- 19 830 795
- GB-A- 1 462 049
- US-A- 3 776 987
- US-A- 3 937 667
- US-A- 4 571 315
- US-A1- 2001 012 816

## Beschreibung

Die Erfindung betrifft kugelförmige Metall-Trägerkatalysatoren, ein Verfahren zu deren Herstellung sowie ein Verfahren zur Hydrierung von Aromaten.

Beim Einsatz von Katalysatoren in Festbettreaktoren führt die Kugelform des Katalysators zu einer gleichmäßigen Packung der Katalysatorschüttung und damit zur Vermeidung von unerwünschten Kanälen.

Die Herstellung von kugelförmigen Katalysatoren ist in der Literatur hinreichend beschrieben. Zahlreiche Beispiele sind für die Herstellung von oxidischen Trägern durch Anwenden der Vertropfung von Hydrosolen in verschiedenen Lösungen geschützt: US-PS 4198318 beschreibt die Herstellung kugelförmiger Al₂O₃-Träger durch Vertropfen eines sauren Hydrosols in eine wässrige Ammoniaklösung in Gegenwart eines nichtionischen oberflächenaktiven Mittels. In der DE 403 5089 wird die Vertropfung mit einer vibrierenden Düsenplatte vorgenommen. Die US 2001/0012816 A1 beschreibt die Vertropfung von Gemischen aus Polysaccharidlösungen mit hydratisierten Al₂O₃-, SiO₂ Al₂O₃-, ZrO₂ Al₂O₃- oder TiO₂ Al₂O₃-Gelen beziehungsweise mit Al₂O₃-, B₂O₃ Al₂O₃- oder B₂O₃ SiO₂ Al₂O₃-Hydraten in eine wässrige Lösung von Ca²⁺-, Al³⁺-, Mg²⁺-, Ba²⁺- oder Sr²⁺-Ionen.

CH 566 809 offenbart ein Verfahren zur Herstellung von Metallverbindungen enthaltenden oder daraus bestehenden Formkörpern oder Partikeln, dadurch gekennzeichnet, dass man aus löslichem Metallsalz, polymer, mit den Metallionen einen Komplex bildender organischer Verbindung und Lösungsmittel eine viskose Lösung herstellt, aus dieser Formkörper oder Partikel bildet und diese mit einer weiteren Lösung behandelt, die eine Ausfällung von unlöslicher Metallverbindung bewirkt.

Ein ähnliches Verfahren wird in der US 3 776 987 beschrieben wobei aber ein Metalloxidsol zum Einsatz kommt.

Alle bislang beschriebenen Vertropfungsverfahren sind jedoch lediglich auf oxidische Träger bezogen. Vertropfungsverfahren zur Herstellung von Metall-Trägerkatalysatoren mit Metallgehalten >10 Masse-%, die nach der Vertropfung, Trocknung und eventueller Calcination reduziert werden müssen, wurden bisher noch nicht beschrieben.

Eine weitere Methode zur Herstellung kugelförmiger Katalysatoren ist das Granulierverfahren, das allerdings nicht zu einheitlichen Kugelgrößen führt. Weitere Nachteile dieses Verfahrens sind die raue Oberfläche der Kugeln sowie die ungleichmäßige Verteilung der Porengrößen über den Kugelquerschnitt.

Ferner können kugelförmige Katalysatoren durch Einsatz von "Sphäronizern" hergestellt werden. Hierbei werden zuvor hergestellte Formlinge auf einer rotierenden Platte zu Kugeln verformt, wie am Beispiel von oxidischen Trägern in WQ 99/58236 beschrieben wird. Bei diesen Verfahren wird die Porosität im starken Maße bei der Verformung zu Extrudaten vorbestimmt und die Einheitlichkeit der Kugelgröße und Kugelform ist zudem unbefriedigend.

Der vorliegenden Erfindung liegt daher das technische Problem zugrunde, hochaktive Metall-Trägerkatalysatoren für Hydrierprozesse bereitzustellen, die eine einheitliche Kugelgröße und Kugelform besitzen sowie eine hohe Metalldispersität, eine hohe Porosität und eine gleichmäßige Verteilung der Porengrößen aufweisen.

Der vorliegenden Erfindung liegt außerdem das technische Problem zugrunde, Verfahren zur Herstellung solcher Katalysatoren bereitzustellen.

Dieses technische Problem wird erfindungsgemäß dadurch gelöst, dass ein Verfahren zur Herstellung geformter kugelförmiger Metall-Trägerkatalysatoren mit Metallgehalten von 10 bis 70 Masse-% bereitgestellt wird, wobei ein in einem flüssigen Medium suspendiertes Gemisch aus mindestens einem gelösten Alginat und mindestens einer Eisen-, Kobalt-, Nickel-, Kupfer- oder Zinkverbindung der Gruppe der Metalloxide, Metallhydroxide, basische Metallcarbonate, Metallhydrogencarbonate, Metallsilikate, Metallzirkonate, Metallaluminate, Metalltitanate, Metallchromite oder Metallalumi-nosilikate in eine Metallsalzlösung getropft wird, deren Metallionen vorzugsweise auch Bestandteil der mindestens einen ausgewählten Metallverbindung sind. Als Metallsalzlösung werden erfindungsgemäß bevorzugt Verbindungen anderer mehrwertiger Kationen, wie Ni²⁺, Cu²⁺ Mg²⁺, Ca²⁺, Sr²⁺, Ba²⁺, Mn²⁺, Al³⁺ und Cr³⁺ eingesetzt werden. Die in dieser Metallsalzlösung aus dem Gemisch erhaltenen kugelförmigen Katalysatorformlinge werden nach einer Verweilzeit von 1 bis 180 Minuten aus dieser abgetrennt, bei Temperaturen von 80 bis 150°C getrocknet und bei Temperaturen von 150 bis 600°C reduziert. Anschließend werden die Katalysatorformlinge bevorzugt in an sich bekannter Weise stabilisiert.

Durch das erfindungsgemäße Verfahren werden kugelförmige Katalysatorformlinge erhalten, die eine sehr einheitliche Kugelform aufweisen. Die mit dem erfindungsgemäßen Verfahren erhaltenen Katalysatoren weisen zusätzlich eine höchst einheitliche Partikelgröße auf.

Gegenüber den durch Verfahren nach dem Stand der Technik erhaltenen Katalysatoren weisen die nach dem erfindungsgemäßen Verfahren erhaltenen Katalysatoren ein deutlich vergrößertes Porenvolumen mit deutlich mehr Makroporen oberhalb 50 nm auf. Erfindungsgemäß führt der hohe Anteil an Makroporen zu einer schnellen Abführung des Reduktionswassers aus dem Katalysatorformling. Ein besonderer Vorteil des erfindungsgemäßen Verfahrens besteht daher auch in den sehr hohen Metalldispersitäten der reduzierten Katalysatoren.

Ein weiterer Vorteil der erfindungsgemäß erhaltenen Katalysatorformlinge besteht in der auch nach der Reduktion hohen mechanischen Festigkeit bei gleichzeitig hohem Porenvolumen.

Durch die Wahl der mindestens einen Eisen-, Kobalt-, Nickel-, Kupfer- oder Zinkverbindung im Gemisch und/oder durch das Feststoffverhältnis dieser Verbindung zu dem mindestens einen zugesetzten Polysaccharid kann die Porenstruktur der erhaltenen erfindungsgemäßen Katalysatoren gezielt eingestellt werden. Erfindungsgemäß bevorzugt sind Feststoffverhältnisse (bezogen auf den Glührückstand) von 4 bis 15, insbesondere von 4,4 bis 8,5. Erfindungsgemäß bevorzugt wird als flüssiges Medium Wasser eingesetzt.

In einer bevorzugten Ausführungsform wird die mechanische Festigkeit der Kugeln durch mindestens einen festen und/oder flüssigen Zuschlagsstoff, der zu der mindestens einen Eisen-, Kobalt-, Nickel-, Kupfer- oder Zinkverbindung, vorzugsweise vor der Vertropfung, zugegeben wird und als Bindemittel wirkt, erhöht. Erfindungsgemäß bevorzugt wird mindestens einer der folgenden Zuschlagstoffe eingesetzt: Tylose, Bentonit, Böhmite, Kaolin, Kieselgel, Methylcellulose, Kieselsol und Wasserglas. Erfindungsgemäß bevorzugt sind Feststoffverhältnisse (bezogen auf den Glührückstand) der mindestens einen Eisen-, Kobalt-, Nickel-, Kupfer- oder Zinkverbindung zu dem mindestens einen Zuschlagstoff von 4 bis 15, insbesondere von 10 bis 12. In einer Variante kann durch Menge und Natur der Zuschlagsstoffe die Porenstruktur der erhaltenen erfindungsgemäßen Katalysatoren gezielt eingestellt werden.

Erfindungsgemäß bevorzugt wird die mechanische Festigkeit des erhaltenen Katalysators durch die Natur und Konzentration des mindestens einen Metallions in der Metallsalzlösung, insbesondere in einer Vertropfungssäule, bestimmt.

Erfindungsgemäß bevorzugt werden für Eisen-Trägerkatalysatoren Eisensalzlösungen, für Kobalt-Trägerkatalysatoren Kobaltsalzlösungen, für NickelTrägerkatalysatoren Nickelsalzlösungen, für Kupfer-Trägerkatalysatoren Kupfersalzlösungen und für Zink-Trägerkatalysatoren Zinksalzlösungen verwendet. Die Metallsalzlösungen können auch andere mehrwertige Metallionen oder Gemische von diesen, insbesondere der Metalle Magnesium, Calcium, Strontium, Barium, Mangan, Aluminium oder Chrom, wie Mg²⁺, Ca²⁺, Sr²⁺, Ba²⁺, Mn²⁺, Al³⁺ oder Cr³⁺, enthalten.

In einer bevorzugten Ausführungsform liegt das mindestens eine Metallsalz in der vorgenannten Metallsalzlösung als Metallnitrat und/oder Metallacetat vor. In einer Variante dieser bevorzugten Ausführungsform liegt das mindestens eine Metallsalz in einer Menge von 0,3 bis 5 Masse-%, bevorzugt von 1 bis 2 Masse-%, in der Metallsalzlösung vor.

In einer weiteren bevorzugten Ausführungsform wird den Metallen Eisen, Kobalt, Nickel, Kupfer und/oder Zink zusätzlich mindestens ein Dotierungselement aus der Gruppe Magnesium, Calcium, Mangan, Molybdän, Chrom, Eisen und Zink in einer Menge von 0,1 bis 5 Masse-% zugesetzt, bevorzugt 1 bis 3 Masse-%. In einer besonders bevorzugten Ausführungsform wird zur Dotierung Mangan in einer Menge von 2 Masse-% eingesetzt.

In einer weiteren bevorzugten Ausführungsform werden die bei der Herstellung des Katalysators in der Metallsalzlösung erhaltenen kugelförmigen Katalysatorformlinge nach einer Verweilzeit von 1 bis 180 Minuten aus dieser abgetrennt und anschließend bei Temperaturen von 80 bis 150°C getrocknet und danach bei Temperaturen von 150 bis 600°C zunächst calciniert bevor sie bei Temperaturen von 150 bis 600°C reduziert werden.

Vorzugsweise wird die Reaktionsführung der Reduktion und Stabilisierung der getrockneten oder geglühten Katalysatoren nach dem Stand der Technik vorgenommen, wie auch exemplarisch in den Ausführungsbeispielen dargelegt.

In diesem Zusammenhang ist ein weiterer Gegenstand der vorliegenden Erfindung ein kugelförmiger Metall-Trägerkatalysator mit den vorgenannten Eigenschaften, der durch eines der erfindungsgemäßen Verfahren erhältlich ist.

In diesem Zusammenhang ist ein weiterer Gegenstand der vorliegenden Erfindung ein Verfahren zur Hydrierung von vorzugsweise substituierten und/oder unsubstituierten Aromaten und/oder Gemische davon, die flüssig oder gasförmig sind, wobei diese Aromaten unter Einsatz des erfindungsgemäßen Metall-Trägerkatalysators, der durch eines der erfindungsgemäßen Verfahren erhältlich ist, hydriert werden.

Im Zusammenhang mit der vorliegenden Erfindung ergeben sich weitere Vorteile und weitere Ausführungsvarianten aus den nachstehenden Ausführungsbeispielen, wobei die erläuterten Merkmale nicht nur in den angegebenen Kombinationen, sondern auch in anderen Kombinationen oder allein anwendbar sein können.

### Beispiel 1 (erfindungsgemäß):

0,6 l Wasser werden in einem Rührbehälter vorgelegt und danach 9 g Natriumalginat zugegeben. Nach vollständigem Lösen des Alginats erfolgt unter Rühren der Zusatz von 5,7 g Siliziumdioxid in Form einer Natronwasserglaslösung mit einer Konzentration von 190 g SiO₂/l Lösung. Die erhaltene Lösung wird 5 min weitergerührt und im Anschluss daran werden 200 g einer durch herkömmliche Füllung hergestellten Nickel-Siliziumdioxid-Metallverbindung mit einem Glührückstand (2 h bei 800°C) von 20 Masse-% (entsprechend 40 g Feststoff bezogen auf Glührückstand) in die Lösung eingetragen.

Das Verhältnis von Metallverbindung (bezogen auf den Glührückstand) und dem verwendeten Alginat (fest) im Gemisch ist 4,4, das Metallverbindung-Zuschlagsstoff-Verhältnis liegt bei 7.

Zur Herstellung einer fließfähigen Suspension wird nun das Gemisch (Slurry) unter Einsatz eines Ultraturraxes für 5 min (500 U/min) homogenisiert und danach kontinuierlich in einen mit Lochschlitzen (3 mm Durchmesser) versehenen Keramiktropfkopf (Volumen ca. 300 ml) gepumpt und daraus vertropft.

Die Vertropfung der Lösung erfolgt in eine Flüssigkeitssäule, die eine wässrige Nickelnitratlösung (1 % Nickel) enthält. Der Abstand zwischen Tropfkopf und Flüssigkeitsoberfläche beträgt etwa 10 cm, das Gesamtlösungsvolumen ist 1,5 l. Unmittelbar nach Eintauchen der Tropfen in die Nickellösung tritt die Gelbildung ein. Die gleichmäßig geformten Kugeln setzen sich am Behälterboden ab. Nach vollständiger Vertropfung wird das so hergestellte Material noch 30 min zur vollständigen Aushärtung in der Lösung belassen.

Danach wird die Lösung dekantiert und die verformten Katalysatorpartikel mit ca. 5 l Reinkondensat gewaschen.

Unmittelbar anschließend erfolgt die Trocknung der Formlinge bei 130°C für 15 h. Nach der Trocknung weist das kugelförmige Katalysatormaterial eine sehr einheitliche Form und Größe auf.

Der getrocknete Katalysator wird danach in einem geeigneten Integralreaktor im Stickstoffstrom bei Temperaturen von ca. 350°C calciniert und nach Umstellung auf Wasserstoff (Gasbelastung ca. 2000 v/vh) für 6 h bei Temperaturen von 400°C reduziert.

Die Stabilisierung des pyrophoren Katalysators erfolgt anschließend unter Verwendung eines Luft/Stickstoff-Gemisches (beginnend mit O₂-Konzentrationen von 0,1 Vol.% bis 2 Vol.%).

Der daraus erhaltene erfindungsgemäße Katalysator enthält 58 % Nickel und besitzt einen Reduktionsgrad von 75 %. Die Nickel-Metalloberfläche (bestimmt durch CO-Chemisorption) beträgt 50 m²/g Katalysator, die mittlere Nickel-Kristallitgröße beträgt 3 nm und die Schüttdichte beträgt 0,5 kg/l.

Der erfindungsgemäße Katalysator weist außerdem eine ausgezeichnete Einheitlichkeit der Partikelgrößenverteilung auf: Der mittlere Durchmesser liegt bei 2,15 mm. Alle Katalysatorpartikel besitzen einen Durchmesser von 2,0 bis 2,3 mm.

In der Tabelle 2 sind die Kenndaten der mechanischen und physikalisch-chemischen Charakterisierung des erfindungsgemäß hergestellten Katalysators zusammengefasst. Daraus ist erkennbar, dass sich der erhaltene Katalysator durch ein extrem hohes Porenvolumen bei gleichzeitig ansprechender Festigkeit der Katalysatorformlinge auszeichnet.

### Beispiel 2 (erfindungsgemäß):

In 0,6 l Wasser werden unter Rühren 6 g Natriumalginat gelöst und anschließend 3,5 g Siliziumoxid in Form einer Kieselsollösung mit einer Konzentration von 498 g SiO₂/l Lösung zugegeben. Nach etwa 5 min erfolgt der Eintrag von 60 g eines sprühgetrockneten pulverförmigen Katalysatorzwischenproduktes mit einem Glührückstand (2 h 800°C) von 75 % (entsprechend 45 g Feststoff bezogen Glührückstand), welches auf an sich bekannte Weise durch gemeinsame Fällung von Nickel, Aluminiumoxid und Siliziumdioxid mit NaOH hergestellt wird.

Das verwendete Pulver weist eine Dichte von ca. 0,3 kg/l und eine mittlere Korngröße von ca. 8 um auf.

Das Verhältnis von Metallverbindung (bezogen auf den Glührückstand) und dem verwendeten Alginat (fest) im Gemisch ist 7,5, das Metallverbindung-Zuschlagsstoff-Verhältnis liegt bei 13.

Nach der Homogenisierung der Suspension mittels Ultraturrax wird die Suspension wie im Beispiel 1 beschrieben in eine wässrige Nickelacetatlösung (5 % Nickel) vertropft. Das vertropfte Material lagert 15 min in der Nickelacetatlösung.

Nach Abtrennung dieser Lösung werden nun die Katalysatorformlinge getrocknet und anschließend ohne Calcination im Wasserstoffstrom bei Temperaturen von 380°C im Wasserstoffstrom reduziert.

Die Stabilisierung des pyrophoren Katalysators erfolgt im Stickstoff/Luft/Kohlendioxid-Gemisch in an sich bekannter Weise. Der CO₂-Gehalt im Gasgemisch beträgt 1 Vol.%.

Der erhaltene erfindungsgemäße Katalysator enthält 55 % Nickel und besitzt einen Reduktionsgrad von 60 %. Die Nickel-Metalloberfläche (bestimmt mittels CO-Chemisorption) beträgt 33 m²/g Katalysator, die Nickel-Kristallitgröße beträgt 2,7 nm und die Schüttdichte beträgt 0,4 kg/l.

Der erhaltene Katalysator weist außerdem eine ausgezeichnete Einheitlichkeit der Partikelgrößenverteilung mit einen mittleren Durchmesser von 2,6 mm auf. Alle Katalysatorteilchen besitzen dabei einen Durchmesser von 2,4 bis 2,8 mm.

Die Ergebnisse der mechanischen und physikalisch-chemischen Charakterisierung des erfindungsgemäßen Katalysators 2 sind ebenfalls in der Tabelle 2 dargestellt. Es ist erkennbar, dass sich der Katalysator ebenfalls durch ein sehr hohes Porenvolumen bei guter Festigkeit der Formlinge auszeichnet.

### Beispiel 3 (erfindungsgemäß):

In 0,6 l Wasser werden unter Rühren 12 g Natriumalginat gelöst und anschließend eine mechanische Mischung, bestehend aus 80 g eines herkömmlich hergestellten pulverförmigen, getrockneten und calcinierten Nickel-Aluminiumoxid-Fällkatalysators und 8 g eines Böhmits (Versal 250 der Firma UOP, Glührückstand 74,7 %, entsprechend 6 g Feststoff bezogen auf Glührückstand, zugegeben. Das oxidische Katalysatorausgangsmaterial weist eine Schüttdichte von 0,8 kg/l und eine mittlere Korngröße von 10 µm auf.

Das Verhältnis von Metallverbindung (bezogen auf den Glührückstand) und dem verwendeten Alginat (fest) im Gemisch ist 6, das Metallverbindung-Zuschlagsstoff-Verhältnis liegt bei 12.

Die Herstellung der vertropfbaren Katalysatorsuspension erfolgt auf dem in Beispiel 1 angegebenen Weg. Die Vertropfung der Lösung wird in eine 3%ige Nickelnitratlösung vorgenommen. Die vertropften Katalysatorpartikel weisen dabei eine sehr einheitliche Form und Größe auf.

Nach Trocknung und Calcination des genommenen Katalysatormaterials wird die Reduktion bei 420°C im Wasserstoffstrom durchgeführt. Die Stabilisierung der Katalysatorkugeln erfolgt unter den im Beispiel 1 geschilderten Bedingungen.

Der erhaltene erfindungsgemäße Katalysator enthält 65 % Nickel und besitzt einen Reduktionsgrad von 70 %. Die Nickel-Metalloberfläche (bestimmt mittels CO-Chemisorption) beträgt 28 m²/g Katalysator, die Nickel-Kristallitgröße beträgt 3,7 nm und die Schüttdichte beträgt 0,75 kg/l.

Der erhaltene Katalysator weist außerdem eine gute Einheitlichkeit der Partikelgrößenverteilung auf. Der mittlere Partikeldurchmesser beträgt 2 mm. Alle Katalysatorteilchen besitzen einen Durchmesser von 1,9 bis 2,2 mm.

Die Ergebnisse der mechanischen und physikalisch-chemischen Charakterisierung des erfindungsgemäßen Katalysators 3 sind in der Tabelle 2 dargestellt.

### Beispiel 4 (erfindungsgemäß):

In 0,6 l Wasser werden unter Rühren 9 g Natriumalginat gelöst und anschließend 8 g eines handelsüblichen Kieselgels mit einem Glührückstand von 90 % zugegeben. Danach erfolgt die Dosierung von 80 g eines pulverförmigen, getrockneten und calcinierten Kobalt-Mangan-Titanoxid-Fällkatalysators mit einem Glührückstand von ca. 95 %, entsprechend 76,5 g Feststoff bezogen auf Glührückstand. Das pulverförmige Ausgangsmaterial liegt hinsichtlich der Korngröße komplett unter 63 µm, die Dichte beträgt 0,8 kg/l.

Das Verhältnis von Metallverbindung (bezogen auf den Glührückstand) und dem verwendeten Alginat (fest) ist 8,5. Das Metallverbindung-Zuschlags-Verhältnis liegt bei 10,6.

Die Herstellung der vertropfbaren Katalysatorsuspension erfolgt auf dem in Beispiel 1 angegebenen Weg. Die Vertropfung der Katalysatorslurry wird in eine 1,5%igen Calciumnitratlösung vorgenommen. Die vertropften Teilchen weisen dabei eine gleichmäßige und runde Form auf.

Nach Trocknung und Calcination des gewonnenen Katalysatormaterials besitzt es einen Kobaltgehalt von 30 % und einen Mangangehalt von 2 %.

Der erfindungsgemäße Katalysator wird vor seiner katalytischen Beurteilung für die Hydrierung von substituierten Aromaten bei Temperaturen von 400°C reduziert und auf an sich übliche Weise stabilisiert.

Er weist bei kleiner Partikelgrößenverteilung eine mittlere Partikelgröße 2,1 mm auf. Alle Katalysatorpartikel haben dabei einen Durchmesser von 1,8 bis 2,2 mm.

### Beispiel 5 (erfindungsgemäß):

In 0,3 l einer wässrigen Natronwasserglaslösung mit einem SiO₂-Gehalt von 61 g/l Lösung, entsprechend 18,3 g SiO₂, werden unter Rühren 100 g eines sprühgetrockneten Kupfer-Siliziumdioxid-Fällkatalysators mit einem Glührückstand von 73,5 % (entsprechend 73,5 g Feststoff bezogen auf den Glührückstand) zugegeben. Danach erfolgt der Zusatz von 300 g einer 2 %igen Natriumalginatlösung, entsprechend 6 g Alginat.

Das Verhältnis von Metallverbindung (bezogen auf den Glührückstand) und verwendetem Alginat (fest) im Gemisch ist 12,25, das Metallverbindung-Zuschlagsstoff-Verhältnis liegt bei 4.

Nach Homogenisierung der Suspension wird diese unter Rühren auf 60°C erwärmt und ca. 15 min bei dieser Temperatur behandelt. Nach Abkühlung der homogenen Suspension und Nachbehandlung mit dem Ultraturrax erfolgt die Vertropfung in eine 3%ige Kupfernitratlösung. Nach Abschluss der Vertropfung verbleiben die kugelförmigen Katalysatorpartikel noch 10 min in der Kupfernitratlösung.

Nach Abtrennung der Kupfernitratlösung und dem Waschen des gewonnenen Katalysatormaterials erfolgt die Trocknung und Calcination.

Der erhaltene erfindungsgemäße Katalysator weist eine mittlere Partikelgröße von 2 mm auf und zeichnet sich durch eine einheitliche Größe und Form aus.

Im Anschluss an die Calcination wird der Katalysator im Stickstoff/Wasserstoffstrom (2 % H₂) bei Temperaturen von 200°C reduziert und anschließend gemäß Beispiel 1 stabilisiert.

Der Fertigkatalysator enthält 65 % Kupfer und besitzt einen Reduktionsgrad von 70 %. Die mittlere Kupferprimärteilchengröße liegt bei 8,7 nm.

Der erfindungsgemäße Katalysator weist außerdem eine gute Einheitlichkeit der Partikelgrößenverteilung auf. Alle Katalysatorpartikel haben einen Durchmesser von 1,8 bis 2,1 mm, der mittlere Partikeldurchmesser beträgt 1,9 mm und die Schüttdichte beträgt 0,4 kg/l.

### Beispiel 6 (Vergleichskatalysator):

Getrocknetes und gemahlenes Nickel/SiO₂-Ausgangsmaterial mit einer mittleren Korngröße von 10 µm und einer Schüttdichte von 0,7 kg/l wird mit Tylose als Bindemittel gemischt und anschließend in einem Laborkneter unter Zusatz von Kondensatwasser, Salpetersäure und Kieselsollösung peptisiert. Der Tylosezusatz beträgt bezogen auf den Feststoffgehalt des Knetansatzes 2,5 %. Nach einer Knetzeit von 15 min wird der komplette Ansatz in einer Laborstrangpresse mit Abschneidvorrichtung zu 3 mm Vollsträngen verformt.

Die erhaltenen feuchten Extrudate werden nun in einem Laborsphäronizer (Fa. Caleva, Model 120, England) zu Kugeln weiterverarbeitet. Das erhaltene kugelförmige Material wird im Anschluss daran bei 130°C getrocknet und weist das in Tabelle 1 dargestellte Kornspektrum auf.

**Tabelle 1:**

| Korngröße (mm) | Masse-% |
|---|---|
| > 5 | - |
| 4-5 | 0,1 |
| 3-4 | 10,7 |
| 2,5-3,0 | 39,9 |
| 2, 0-2, 5 | 25,8 |
| 1,6-2,0 | 16,1 |
| 1,0-1,6 | 7,4 |
| < 1,0 | - |

Neben dem sehr breiten Kornspektrum besitzen die Katalysatorpartikel eine uneinheitliche und zum Teil ungleichmäßige sphärische Form.

Das erhaltene Katalysatormaterial wird anschließend bei 400°C im Wasserstoffstrom reduziert und unter Standardbedingungen in an sich bekannter Weise stabilisiert.

Der erhaltene Vergleichskatalysator enthält 55 % Nickel und besitzt einen Reduktionsgrad von 75 %. Die Nickel-Metalloberfläche (bestimmt durch CO-Chemisorption) beträgt 30 m²/g Katalysator, die mittlere Nickel-Kristallitgröße beträgt 4,5 nm und die Schüttdichte beträgt 0,8 kg/l.

Der Katalysator zeigt eine breite Partikelgrößenverteilung mit Durchmessern von 2 bis 4 mm.

Weitere Kenndaten der physikalisch-chemischen Charakterisierung des erhaltenen Vergleichskatalysators sind in der Tabelle 2 enthalten. Es ist erkennbar, dass der Vergleichskatalysator ein deutlich geringeres Porenvolumen mit deutlich weniger Makroporen oberhalb 5 nm gegenüber den erfindungsgemäß hergestellten Katalysatoren aufweist.

### Beispiel 7 (Vergleichskatalysator):

Das gemäß Beispiel 4 getrocknete und calcinierte Kobalt-Mangan-Titandioxid-Katalysatorausgangsmaterial wird unter Zusatz von Graphit zu 3 mm x 3 mm Tabletten verformt. Im Anschluss an die Verformung wird das Katalysatormaterial wie im Beispiel 4 beschrieben reduziert und stabilisiert.

Der fertige Katalysator weist eine Schüttdichte von 1,2 kg/l und eine Druckfestigkeit von 35 MPa auf.

Die Ergebnisse der mechanischen und physikalischen Charakterisierung des Vergleichskatalysators sind in der Tabelle 2 zusammengefasst.

### Beispiel 8 (Vergleichskatalysator):

Das im Beispiel 5 verwendete Kupfer-Siliziumdioxid-Katalysatorausgangsmaterial wird bei 350°C calciniert und anschließend unter Zusatz von Tylose, Kondensatwasser und Salpetersäure in einem geeigneten Mischaggregat peptisiert und anschließend zu 1,6 mm Trilobe-Extrudaten verformt. Nach Trocknung und Calcination der Formlinge erfolgt die Reduktion und Stabilisierung des Ausgangsmaterials unter den im Beispiel 5 beschriebenen Bedingungen.

Der fertige Katalysator enthält 70 % Kupfer und besitzt einen Reduktionsgrad von 75 %. Die Schüttdichte liegt bei 0,85 kg/l, die Kupferkristallitgröße bei 9,2 nm.

### Beispiel 9 (Vergleichskatalysator):

Zur katalytischen Beurteilung wird auch ein handelsüblicher kugelförmiger Nickel-Alumosilikat-Katalysator mit folgenden physikalisch-chemischen Kenndaten herangezogen:

| | |
|---|---|
| Nickelgehalt (Masse-%) | 55 |
| Nickelreduktionsgrad (%) | 60 |
| Schüttdichte (kg/l) | 0,95 |
| Mittlerer Partikeldurchmesser (mm) | 2,5 |
| Partikeldurchmesserbereich (mm) | 1,6-4,7 |
| Nickelkristallitgröße (nm) | 5,1 |

Neben der sehr breiten Partikelgrößenverteilung weist dieser Katalysator eine sehr uneinheitliche sphärische Form auf. So enthält er neben kugelförmigen Teilchen unterschiedlicher Größe auch Extrudate und Extrudatbruchstücke.

**Tabelle 2:**

| Physikalisch-chemische und mechanische Kenndaten der erfindungsgemäß hergestellten Katalysatoren und der Vergleichskatalysatoren | | | | |
|---|---|---|---|---|
| Katalysator | Schüttdichte (kg/l) | Abrieb^{*} (%) | Gesamtporenvolumen (cm³) | Porenvolumen >50 nm (cm³/g) |
| 1 (erfind.) | 0,5 | 0,8 | 0,84 | 0,52 |
| 2 (erfind.) | 0,4 | 1,0 | 0,95 | 0,64 |
| 3 (erfind.) | 0,75 | 0,7 | 0,60 | 0,29 |
| 4 (erfind.) | 0,5 | 0,3 | 0,45 | 0,29 |
| 5 (erfind.) | 0,4 | 1,2 | 0,82 | 0,58 |
| 6 (Vergl.) | 0,8 | 0,55 | 0,30 | 0,02 |
| 7 (Vergl.) | 1,2 | 0,5 | 0,27 | 0,14 |
| 8 (Vergl.) | 0,85 | 1,2 | 0,30 | 0,07 |
| 9 (Vergl.) | 0,95 | 0,8 | 0,35 | 0,04 |

| | | | | |
|---|---|---|---|---|
| ^{*} Standardabriebtest (Rolltest, 25g Katalysator, 40 U/min, 30min) | | | | |

Beispiel 10 (Hydrierung von Aromaten mit NickelTrägerkatalysatoren):

Für die katalytische Charakterisierung der NickelTrägerkatalysatoren nach Beispiel 1 bis 3 im Vergleich zu den Vergleichskatalysatoren nach Beispiel 6 und 9 wird die Aromaten-Hydrierung von Kerosin im Festbettverfahren mittels Integralströmungsreaktor (Innendurchmesser: 25 mm) eingesetzt.

Das eingebaute Katalysatorvolumen beträgt 50 ml. Die 50 ml Katalysatorvolumen werden jeweils in 10 Portionen mit 10 Portionen von SiC im Volumenverhältnis von 1:1 eingebaut.

Vor der katalytischen Reaktion werden die Katalysatoren im Wasserstoffstrom (50 l/h) über einen Zeitraum von 4 h bei 250°C reaktiviert. Als Feed wird ein Kerosin mit einem Aromatengehalt von 18 Masse-% und einem Schwefelgehalt von 1,1 ppm eingesetzt. Die Reaktionsbedingungen sind:

| | | |
|---|---|---|
| Reaktionsdruck | 30 bar | |
| Reaktionstemperatur | 85°C | 100°C |
| Reaktionszeit | 40 h | 80 h |
| LHSV | 1,3 ml/ml·h | |
| Gas-Produkt-Volumenverhältnis H₂ / Kerosin | 400:l | |

Die Ergebnisse sind in der Tabelle 3 zusammengestellt.

**Tabelle 3:**

| Katalysator | Reaktionstemperatur 85 °C | Reaktionstemperatur 100°C |
|---|---|---|
| | Aromaten-Anteil im Reaktionsprodukt (ppm) | Aromaten-Anteil im Reaktionsprodukt (ppm) |
| 1 (erfind.) | 1600 | 150 |
| 2 (erfind.) | 1750 | 160 |
| 3 (erfind.) | 1550 | 152 |
| 6 (Vergl.) | 2640 | 259 |
| 9 (Vergl.) | 3105 | 298 |

Ein Vergleich der Messergebnisse der katalytischen Hydrierungen verdeutlicht den Vorteil der erfindungsgemäßen Katalysatoren: Der Hydriergrad bzw. Abbau der Aromaten ist bei den erfindungsgemäßen Katalysatoren signifikant größer als bei den herkömmlichen Katalysatoren.

### Beispiel 11 (Hydrierung von Aromaten mit Kobalt-Mangan-Trägerkatalysatoren):

Der Kobalt-Mangan-Titandioxid-Katalysator (Beispiel 4) wird in der Hydrierung von Isocamphylbrenzcatechin zu Sandelalkoholen katalytisch getestet. Als Vergleichskatalysator wird ein verpillter Katalysator (Beispiel 7) der gleichen Zusammensetzung getestet.

Die katalytische Prüfung wird im Festbettverfahren mittels Integralströmungsreaktor (Innendurchmesser: 25 mm) vorgenommen. Es wird ein Katalysatorvolumen von 50 ml eingesetzt. Das 50 ml Katalysatorvolumen wird jeweils in 10 Portionen mit jeweils 10 Portionen SiC im Volumenverhältnis von 1:1 eingebaut.

Vor der katalytischen Reaktion werden die Katalysatoren im Wasserstoffstrom (50 l/h) über einen Zeitraum von 3 h bei 300 °C reaktiviert. Als Feed wird ein Gemisch von Isocamphylbrenzcatechin und Cyclohexanol (1:1) eingesetzt. Die weiteren Reaktionsbedingungen sind:

| | |
|---|---|
| Reaktionsdruck | 70 bar |
| Reaktionstemperatur | 220 °C |
| Reaktionszeit | 50 h |
| LHSV (Isocamphylbrenzcatechin) | 0,5 ml/ml·h. |
| Gas-Produkt-Volumenverhältnis H₂ / Isocamphylbrenzcatechin | 4000:l |

Die katalytischen Messergebnisse sind in Tabelle 4 angeführt.

**Tabelle 4:**

| Katalysator | Katalysatorform Porenradien | Ausbeute an Sandelalkohol (%) | Ausbeute an Kohlenwasserstoffen (%) |
|---|---|---|---|
| 4 (erfind.) | Kugel 2,1 mm | 82,1 | 7,9 |
| 7 (Vergl.) | Tablette 3mm | 68,9 | 10, 8 |

Die Ergebnisse zeigen, dass der erfindungsgemäße Katalysator bei gleicher chemischer Zusammensetzung eine höhere Aktivität und eine höhere Selektivität aufweist als der strangförmige Katalysator.

### Beispiel 12 (Hydrierung von Aromaten mit Kupfer-Trägerkatalysatoren):

Mit dem gleichen Reaktorsystem wie in Beispiel 11 wird auch die katalytische Charakterisierung des Kupfer-Siliziumdioxid-Katalysators (Beispiel 5) in der Acetophenon-Hydrierung vorgenommen. Als Vergleichskatalysator dient der Katalysator aus Beispiel 8. Als Feed wird ein Gemisch von 70 Masse-% Acetophenon und 30 Masse-% Methyl-Phenyl-Carbinol eingesetzt. Die weiteren Reaktionsbedingungen sind:

| | |
|---|---|
| Reaktionsdruck | 20 bar |
| Reaktionstemperatur | 80°C |
| Reaktionszeit | 40 h |
| LHSV | 0,5 ml/ml·h |
| Gas-Produkt-Volumenverhältnis H₂ / Feed | 250:l |

Die katalytischen Messergebnisse sind in Tabelle 5 angeführt.

**Tabelle 5:**

| Katalysator | Katalysatorform | Ausbeute an Methyl-Phenyl-Carbinol (%) |
|---|---|---|
| 5 (erfind.) | Kugel | 76,4 |
| 8 (Vergl.) | Trilobe-Extrudat | 66,9 |

Die Ergebnisse zeigen, dass der erfindungsgemäße Katalysator bei gleicher chemischer Zusammensetzung eine höhere katalytische Aktivität aufweist als der Vergleichskatalysator

## Patentansprüche

1. Verfahren zur Herstellung geformter kugelförmiger Metall-Trägerkatalysatoren mit Metallgehalten von 10 bis 70 Masse-%, wobei
- ein in einem flüssigen Medium suspendiertes Gemisch aus einem gelösten Alginat und mindestens einer Metallverbindung, ausgewählt aus der Gruppe bestehend aus
a) Metalloxiden,
b) Metallhydroxiden,
c) basischen Metallcarbonaten,
d) Metallyhdrogencarbonaten,
e) Metallsilikaten,
f) Metallzirkonaten,
g) Metallaluminaten,
h) Metalltitanaten,
i) Metallchromiten und
j) Metallaluminosilikaten
der Metalle Eisen, Kobalt, Nickel, Kupfer oder Zink in eine Metallsalzlösung getropft wird,
- die in der Metallsalzlösung erhaltenen Katalysatorkugeln nach einer Verweilzeit von 1 bis 180 Minuten aus dieser abgetrennt,
- bei Temperaturen von 80 bis 150°C getrocknet und
- bei Temperaturen von 150 bis 600°C reduziert werden.

2. Verfahren nach Anspruch 1, wobei die Metallsalzlösung aus mindestens einem Metallion der mindestens einen ausgewählten Metallverbindung besteht.

3. Verfahren nach Anspruch 1 oder 2, wobei die Metallsalzlösung aus mindestens einem mehrwertigen Metallion ausgewählt aus der Gruppe bestehend aus Ni²⁺, Cu²⁺, Mg²⁺, Ca²⁺, Sr²⁺, Ba²⁺, Mn²⁺, Al³⁺ und Cr³⁺ besteht.

4. Verfahren nach einem der Ansprüche 1 bis 3, wobei die Metallionen-Konzentration 0,3 bis 5 Masse-%, vorzugsweise 1 bis 2 Masse-%, beträgt.

5. Verfahren nach einem der Ansprüche 1 bis 4, wobei die Metallsalzlösung als Anionen Nitrat und/oder Acetat enthält.

6. Verfahren nach einem der Ansprüche 1 bis 5, wobei bei dem Gemisch mindestens ein Zuschlagstoff zugefügt wird, der als Binder fungiert.

7. Verfahren nach Anspruch 6, wobei der Zuschlagstoff ausgewählt ist aus der Gruppe bestehend aus Tylose, Bentonit, Böhmit, Kaolin, Kieselgel, Kieselsol, Methylcellulose und Wasserglas.

8. Verfahren nach Anspruch 6 oder 7, wobei das Feststoffverhältnis, bezogen auf den Glührückstand, der im Gemisch befindlichen mindestens einen Metallverbindung und dem mindestens einen Zuschlagstoff 4 bis 15 beträgt.

9. Verfahren nach einem der vorangehenden Ansprüche, wobei das Feststoffverhältnis, bezogen auf den Glührückstand, der im Gemisch befindlichen mindestens einen Metallverbindung und dem Alginat 4 bis 15 beträgt.

10. Verfahren nach einem der vorangehenden Ansprüche, wobei dem Gemisch ein oder mehrere Dotierungselemente von 0,1 bis 5 Masse-% zugesetzt werden.

11. Verfahren nach Anspruch 10, wobei die Dotierungselemente Mg, Ca, Mn, Mo, Cr, Fe oder Zn sind.

12. Verfahren nach einem der vorangehenden Ansprüche, wobei die erhaltenen Katalysatorkugeln nach der Trocknung und vor der Reduktion bei Temperaturen von 150 bis 600°C calciniert werden.

13. Kugelförmiger Metall-Trägerkatalysator mit einem Metallgehalt von 10 bis 70 Masse-%, erhältlich durch ein Verfahren nach einem der Ansprüche 1 bis 12.

14. Verfahren zur Hydrierung von Aromaten, wobei die Aromaten unter Einsatz eines kugelförmigen Metall-Trägerkatalysators mit einem Metallgehalt von 10 bis 70 Masse-%, erhältlich durch ein Verfahren nach einem der Ansprüche 1 bis 12, hydriert werden.

## Claims

1. A process for the production of formed spherical metal supported catalysts having a metal content of 10 to 70% by mass, wherein:
- a mixture, suspended in a liquid medium, of a dissolved alginate and at least one metal compound selected from the group consisting of:
a) metal oxides;
b) metal hydroxides;
c) basic metal carbonates;
d) metal hydrogencarbonates;
e) metal silicates;
f) metal zirconates;
g) metal aluminates;
h) metal titanates;
i) metal chromites; and
j) metal aluminosilicates;
of the metals iron, cobalt, nickel, copper or zinc is dropped into a metal salt solution;
- after a standing time of 1 to 180 minutes, the catalyst spheres obtained in the metal salt solution are separated from the latter;
- the catalyst spheres are dried at a temperature of 80 to 150°C and
- the catalyst spheres are reduced at a temperature of 150 to 600°C.

2. A process according to claim 1, wherein the metal salt solution comprises at least one metal ion of the at least one metal compound selected.

3. A process according to claim 1 or claim 2, wherein the metal salt solution consists of at least one polyvalent metal ion selected from the group consisting of Ni²⁺, Cu²⁺, Mg²⁺, Ca²⁺, Sr²⁺, Ba²⁺, Mn²⁺, Al³⁺ and Cr³⁺_{.}

4. A process according to any one of claims 1 to 3, wherein the concentration of metal ions is 0.3 to 5% by mass, preferably 1 to 2% by mass.

5. A process according to any one of claims 1 to 4, wherein the metal salt solution contains nitrate and/or acetate as anions.

6. A process according to any one of claims 1 to 5, wherein at least one additive acting as a binder is added to the mixture.

7. A process according to claim 6, wherein the additive is selected from the group consisting of tylose, bentonite, boehmite, kaolin, silica gel, silica sol, methyl cellulose and water glass.

8. A process according to claim 6 or claim 7, wherein the solids ratio, relative to the ignition residue, of the at least one metal compound present in the mixture to the at least one additive is 4-15.

9. A process according to any one of the preceding claims, wherein the solids ratio, relative to the ignition residue, of the at least one metal compound present in the mixture to the alginate is 4-15.

10. A process according to any one of the preceding claims, wherein one or more doping elements of 0.1 to 5% by mass is/are added to the mixture.

11. A process according to claim 10, wherein the doping elements are Mg, Ca, Mn, Mo, Cr, Fe or Zn.

12. A process according to any one of the preceding claims, wherein, after drying and before reduction, the catalyst spheres obtained are calcined at temperatures of 150 to 600°C.

13. A spherical metal supported catalyst having a metal content of 10 to 70% by mass, which is obtainable by a process according to any one of the claims 1 to 12.

14. A process for the hydrogenation of aromatic substances, wherein the aromatic substances are hydrogenated using a spherical metal supported catalyst having a metal content of 10 to 70% by mass which is obtainable by a process according to any one of claims 1 to 12.

## Revendications

1. Procédé de préparation de catalyseurs à composante métallique sur support sphérique, présentant des teneurs en métal de 10 à 70% en masse, dans lequel
- un mélange mis en suspension dans un milieu liquide et composé d'un alginate dissous et d'au moins un composé métallique choisi dans le groupe formé par
a) des oxydes métalliques,
b) des hydroxydes métalliques,
c) des carbonates métalliques basiques,
d) des hydrogénocarbonates métalliques,
e) des silicates métalliques,
f) des zirconates métalliques,
g) des aluminates métalliques,
h) des titanates métalliques,
i) des chromites métalliques et
j) des aluminosilicates métalliques
des métaux fer, cobalt, nickel, cuivre ou zinc est ajouté goutte à goutte à une solution de sel métallique,
- les sphères de catalyseur obtenues dans la solution de sel métallique sont séparées de celle-ci après un temps de séjour de 1 à 180 minutes,
- séchées à des températures de 80 à 150°C et
- réduites à des températures de 150 à 600°C.

2. Procédé suivant la revendication 1, dans lequel la solution de sel métallique consiste en au moins un ion métallique du au moins un composé métallique choisi.

3. Procédé suivant la revendication 1 ou 2, dans lequel la solution de sel métallique consiste en au moins un ion métallique multivalent choisi dans le groupe formé par Ni²⁺, Cu²⁺, Mg²⁺, Ca²⁺, Sr²⁺, Ba²⁺, Mn²⁺, Al³⁺ et Cr³⁺.

4. Procédé suivant l'une quelconque des revendications 1 à 3, dans lequel la concentration des ions métalliques est de 0,3 à 5% en masse, de préférence de 1 à 2% en masse.

5. Procédé suivant l'une quelconque des revendications 1 à 4, dans lequel la solution de sel métallique contient en tant qu'anions du nitrate et / ou de l'acétate.

6. Procédé suivant l'une quelconque des revendications 1 à 5, dans lequel le mélange est additionné d'au moins un additif faisant fonction de liant.

7. Procédé suivant la revendication 6, dans lequel l'additif est choisi dans le groupe formé par la tylose, la bentonite, la boehmite, le kaolin, le gel de silice, un sol de silice, la méthylcellulose et le verre soluble.

8. Procédé suivant la revendication 6 ou 7, dans lequel la proportion de solide, par rapport au résidu sec, du au moins un composé métallique et du au moins un additif contenus dans le mélange est de 4 à 15.

9. Procédé suivant l'une quelconque des revendications qui précèdent, dans lequel la proportion de solide, par rapport au résidu sec, du au moins un composé métallique et de l'alginate contenus dans le mélange est de 4 à 15.

10. Procédé suivant l'une quelconque des revendications qui précèdent, dans lequel le mélange est additionné d'un ou plusieurs éléments dopants à raison de 0,1 à 5% en masse.

11. Procédé suivant la revendication 10, dans lequel les éléments dopants sont du Mg, du Ca, du Mn, du Mo, du Cr, du Fe ou du Zn.

12. Procédé suivant l'une quelconque des revendications qui précèdent, dans lequel les sphères de catalyseur obtenues sont, après le séchage et avant la réduction, calcinées à des températures de 150 à 600°C.

13. Catalyseur façonné à composant métallique sur support sphérique ayant une teneur en métal de 10 à 70% en masse, que l'on peut obtenir par un procédé suivant l'une des revendications 1 à 12.

14. Procédé d'hydrogénation d'aromatiques, dans lequel les aromatiques sont hydrogénés avec mise en oeuvre d'un catalyseur à composante métallique sur support sphérique ayant une teneur en métal de 10 à 70% en masse, que l'on peut obtenir par un procédé suivant l'une quelconque des revendications 1 à 12.
